# EUROPEAN PATENT SPECIFICATION

(11) **EP 2 753 303 B1**
(45) Date of publication and mention of the grant of the patent: **04.04.2018**
(21) Application number: 12759873.8
(22) Date of filing: 07.09.2012
(51) Int. Cl.: A61K 9/00, A61K 31/485, A61K 47/34

(54) **SILICONE COATED IMPLANTS**
SILIKONUMMANTELTE IMPLANTATE
DES IMPLANTS ENROBÉS DE SILICONE

(30) Priority: 09.09.2011 US 201161533131 P
(43) Date of publication of application: 16.07.2014
(73) Proprietor: Axxia Pharmaceuticals, Llc, Bel Air, MD 21015-1717 (US)
(72) Inventor: HOLL, Richard, J., Lone Jack, MO 64070 (US); HARTMAN, Katherine, Kansas City, MO 64154 (US); GROSSMAN, Stuart, A., Towson, MD 21287 (US); POLLOCK, Wayne, C., Lincoln University, PA 19352 (US)
(74) Representative: Hamer, Christopher K.
(86) International application number: PCT/US2012/054176
(87) International publication number: WO 2013/036775

(56) References cited:
- EP-A2- 0 009 410
- WO-A2-2010/120389
- US-A1- 2009 208 540
- US-A1- 2010 303 883

## Description

### Field

The subject matter disclosed herein relates to implants for delivery of therapeutic agents such as opioids, and the manufacture and uses of such implants. In particular, the subject invention relates to a subcutaneous drug delivery system comprising a biocompatible thermoplastic elastomeric polymer matrix, a therapeutic agent embedded homogeneously in said matrix, and a biocompatible drug impermeable cross-linked silicone polymer coating said matrix. Unexpectedly, silicone-based adhesives or dispersions can be used as the coating in the construction of the subcutaneous drug delivery system.

### Background

U.S. patents 5,633,000, 5,858,388, and 6,126,956 to Grossman et al. relate to drug delivery systems containing an active agent such as an opioid. These implants have geometry such that the release of the active agent is continuous over extended periods of time. The patents also relate to the manufacture and various uses of the implants. The polymeric implant delivery system described in Grossman et al, discloses a blend of the active compound with Elvax 40W (EVA) when fabricated. The thickness, diameter and central channel surface area provide the release kinetics and blood level required for therapeutic benefit. Grossman et al teach a solvent based process for producing both the internal drug reservoir matrix as well as the drug impermeable external coating e.g. polymethyl methacrylate. Polymethyl methacrylate is a crystalline material.

Solvent-processing of polymethyl methacrylate typically results in a brittle, crystalline coating that is susceptible to cracking during processing and to impact fracture in the finished product.

US patent application 2010/0303883 discloses using thermoplastic polymers as the coating material either applied through solvent-processing techniques or prepared as melt-processed (extruded) thin-films prior to manufacturing the subcutaneous drug delivery system.

WO 2010/120389 relates to delivery systems comprising a thermoplastic elastomer matrix, a therapeutic agent, and a therapeutic agent impermeable thermoplastic polymer coating.

### Summary

According to certain aspects, a subcutaneous delivery system is provided comprising:
i) a biocompatible thermoplastic elastomer matrix,
ii) a therapeutic agent dispersed homogeneously in said matrix, and
iii) a biocompatible therapeutic agent impermeable cross-linked silicone polymer coating said matrix,
wherein the delivery system has a geometry such that there is an external coated wall and an internal uncoated wall forming an opening for release of said therapeutic agent, and the distance between the uncoated wall and the coated wall opposite the uncoated wall is substantially constant throughout the delivery system.

Further aspects provide a subcutaneous delivery system as described in Claim 13, a subcutaneous delivery system for use as described in Claim 14, and a method as described in Claims 15, 19 or 20.

### Brief Description of Drawings

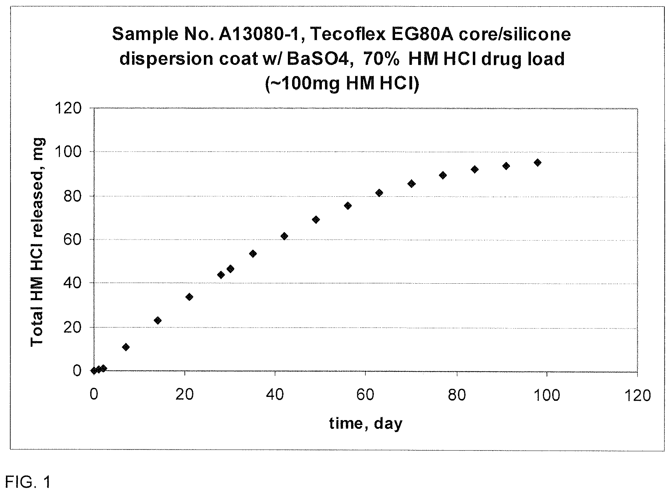

FIG. 1 is a graph showing the mean hydromorphone release results for coated drug reservoir matrices processed as described in Example 4 below. All matrices contain approximately 70% w/w hydromorphone HCI. A center hole was punched with 19G stainless steel tubing. Percent hydromorphone released determined from samples sizes of n=6.

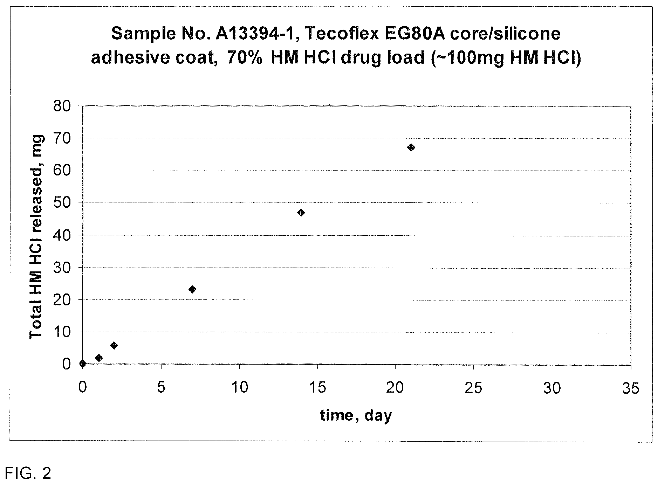

FIG. 2 is a graph showing mean hydromorphone release results for coated drug reservoir matrices processed as described in Example 5 below. All matrices contain approximately 70% w/w hydromorphone HCl. A center hole was cored with 19G stainless steel tubing using a high-speed rotary tool. Percent hydromorphone released determined from samples sizes of n=3.

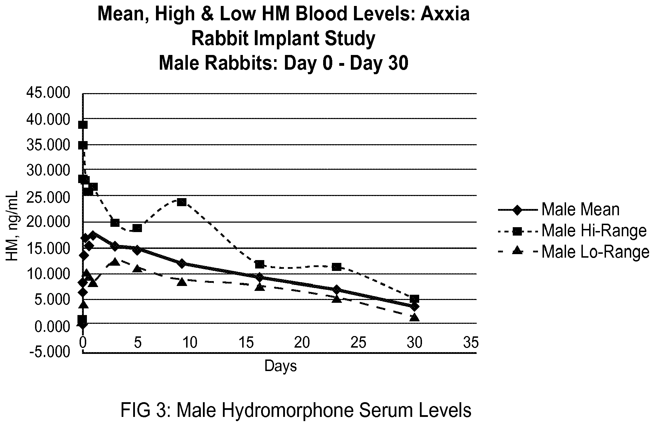

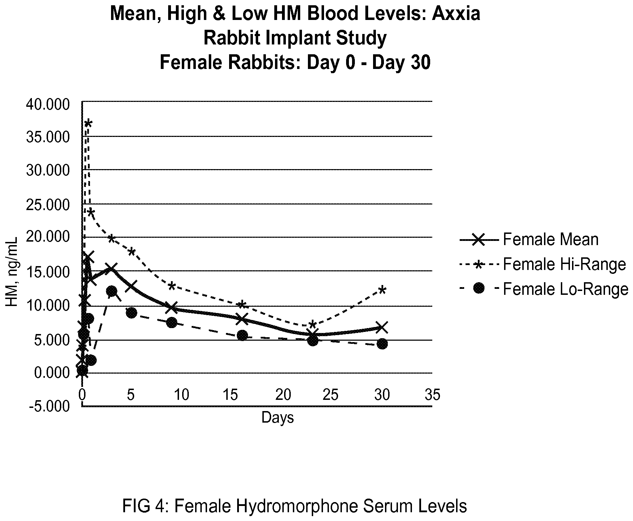

FIGS. 3 and 4 are graphs of hydromorphone serum levels as a function of days post-implantation for male and female rabbits, respectively, according to Example 6 below.

### Detailed Description

Embodiments as disclosed herein relate to a subcutaneous drug delivery system comprising a biocompatible thermoplastic polymer matrix, a therapeutic agent embedded homogeneously in said matrix, and a biocompatible drug impermeable cross-linked silicone polymer coating said matrix, wherein said delivery system has a geometry such that there is an external coated wall and an internal uncoated wall (or channel) forming an opening for release of said therapeutic agent, and the distance between the uncoated wall and the coated wall opposite the uncoated wall is substantially constant throughout the delivery system. The invention also relates to the methods of producing and the use of such delivery systems.

A cross-linked polymer coating, specifically silicone-based adhesives or dispersions, can be used as the coating matrix in the construction of the subcutaneous drug delivery system. It is unexpected that a silicone adhesive, typically used as an adhesive construction aid, can function as the integral component of the biocompatible impermeable cross-link polymer coating.

In addition, the method of manufacture of the implant is very simple in that a single processing step for example, by using web-coating techniques known to be used in thin-film and transdermal device manufacturing, can be used to manufacture the subcutaneous drug delivery system.

Some embodiments relate to implant devices that permit controlled release of a therapeutic agent by subcutaneous implant. The devices provide burst free systemic delivery with near constant release of an active agent for a long duration, i.e. 2 weeks, 4 weeks, 8 weeks, 12 weeks, 16 weeks or 6 months. In specific embodiments of the device, more than one drug can be delivered where the delivery of both drugs is systemic, or the delivery of one drug is systemic without burst while the delivery of the other is local with or without burst. "Near constant" release is defined as a plus or minus five fold (500%), advantageously a two fold (200%), most advantageously a single fold (100%) variation in the target delivery rate (in vivo or in vitro).

The geometry, manufacture and use of implants are disclosed in commonly owned US Patent 5,858,388, and WO 2010/120398 . The implant is advantageously cylindrical in shape. The cylindrical implant is 5-100 mm in diameter and 1-20 mm in height. A single 50 micron-3 mm diameter circular opening extends along the axis of the cylinder creating an internal cylindrical uncoated area through the drug is released. For treatment of cancer pain, with a drug such as hydromorphone, implants are designed to produce drug release rate from 0.1 to 25 mg/hr, advantageously 0.1-10 mg/hr. The thickness (height), diameter and central channel surface area provide the release kinetics and blood level required for therapeutic benefit. In a new embodiment, one or more openings are added to the perimeter wall of cylindrical, e.g. disk implants.

Polymeric drug delivery devices in the form of a subcutaneous implant for reservoiring and controlled steady state release of therapeutic agents such as opioids including hydromorphone, can utilize several categories of resins for:
i) the drug reservoir controlled release matrix, and/or
ii) the drug impermeable coating

The coating, a principal purpose of which is to restrict the release of drug to the surface area of uncoated polymer in the central channel, allows uniform controlled flux with no burst effect. The coating is a significant factor in preventing possible leakage of the active opioid (or other drug) and a potentially uncontrolled and lethal burst effect while the implant is in use. In the subject invention, the coating includes a silicone polymer.

### Core Matrix

### Plastic Resins

Examples of plastic resins useful for i) the drug reservoir matrix and ii) the impermeable coating include:

### Unmodified Homopolymers

Low-density polyethylene
Linear low-density polyethylene
Amorphous polypropylene
Polyisobutylene

### Copolymers

Especially important are copolymers of ethylene.
Ethylene Vinyl Acetate (EVA) up to 40% VA content
Ethyl Acrylate (EAA). Ethylene Acrylic Acid resins
Ethylene Methacrylate (EMA)
Ethylene ethyl acrylates (EEA)
Ethylene butyl acrylate

### Thermoplastic Elastomers (TPEs)

Thermoplastic elastomers such as i) thermoplastic polyurethanes, ii) thermoplastic copolyesters, and iii) thermoplastic polyamides are useful in the embodiments of the subject invention.

Thermoplastic Polyurethanes with PEG, PPG and PTMEG glycol soft segments may be employed, including but not limited to resins based on:
Toluene Diisocyanate (TDI)
Methylene diisocyanate (MDI)
Polymeric isocyanates (PMDI)
Hydrogenated methylene diisocyanate

Thermoplastic Copolyesters e.g. HYTREL® thermoplastic polyester elastomer with PEG, PPG and PTMEG glycol soft segments

Thermoplastic Polyether block amides with PEG, PPG and PTMEG soft segments

### Biodegradable Polymers

Biodegradable polymers such as polyesters, polyether-esters, poly(ortho-esters), poly(amino acids), polyanhydrides, polyamides, polyphophazenes, polyphosphoesters, and copolymers therein known to ones skilled in the art. Especially preferred for certain embodiments disclosed herein are biodegradable polymers possessing degradation rates significantly slower than the release rate of therapeutic agent including but not limited to:
Polycaprolactone
Poly(L-lactide)
Poly(DL-lactide)
Poly(L-lactide-co-glycolide) 85:15 ratio
Poly(L-lactide-co-caprolactone) 70:30 ratio
Poly(dioxanone)
Poly(glycolide-co-trimethylene carbonate)

Release kinetics from a melt blended and extruded polymeric matrix are a function of:
- the chemical structure and aqueous solubility and polymer solubility of the drug component(s),
- drug particle size, which advantageously ranges between 25 and 250 microns for opiates.
- drug loading (the amount of drug added to, blended and compounded into the thermoplastic polymer component of the formulation), advantageously 50%-80%,
- the polymer types, polymer morphology (Tg), hydrophilic properties of the polymeric matrix,
- additives including excipients and plasticizers, and importantly
- the proper balance of physical interconnectivity (channels leading into and out of the polymeric reservoir component) and hydrophilic properties of the polymeric matrix such that the channels allow body fluids to enter the matrix through the exposed surface of the central channel and gain access to particles of active drug dispersed within the core/reservoir component of the implant.

Interconnective porosity within the polymeric/drug matrix is important to the functionality of the implant. There must be multiple interconnecting physical paths from the exposed surface of the central channel into and throughout the core component. These interconnecting paths are one of the functional properties of the polymer which allow body fluids to access the soluble drug component reservoired in the matrix while allowing the solvated drug to exit the matrix and enter circulation.

Another functional property determining drug diffusivity is the hydrophilic nature of the polymer. Depending on the solubility of the drug in the soft segment, a portion of the active agent goes into solution in the polymer while the remaining loading is suspended in the matrix. The polymeric matrix is selected to optimize and control the solubility of the active agent, e.g. hydromorphone HCI, within the polymer itself. Given that hydromorphone HCI is a highly water soluble compound, the polymer must have a high amorphous or soft section component which is hydrophilic in nature. This raises the water content in the polymer and also increases the solubility of the drug in the polymer as well as the diffusivity of the drug out of the polymer into the body fluids surrounding the implant.

The release kinetics as well as the therapeutic functionality of the device are dependent upon the design and selection of a polymeric reservoir which has the following properties:
- Ability to hold up to 80%, e.g. 50-80% by weight of the active agent, e.g. hydromorphone HCI.
- An amorphous, hydrophilic, soft segment -for the thermoplastic elastomercontent of 30-80% of the weight of the thermoplastic elastomer (i.e. 30-80% polyethylene glycol (PEG), polypropylene glycol (PPG), or poly tetramethylene-ethylene glycol (PTMEG)) - this insures controlled solubility of the active agent e.g. hydromorphone, within the amorphous or soft segment of the polymer, and controlled diffusivity out of the polymer and into body fluids. Solubility and diffusivity (a direct function of the chemical composition of the reservoir polymer) are important issues in the functionality of this delivery system.
- A hard segment-for the thermoplastic elastomer--an isocyanate (for polyurethane), polyester (for copolyesters), or polyamide (for polyether block amides) of 20-70%, balanced in content with the soft segment in such a way that a portion (approximately 50%) of the active drug is in solution with the polymer while the remaining portion of the drug is dispersed (not in solution). The functional significance of this design is that the active drug in polymer solution delivers the substance by diffusion into systemic circulation.
- A hard segment-- for the thermoplastic elastomer --an isocyanate (for polyurethane), polyester (for copolyesters) or polyamide (for polyether block amides) that imparts sufficient stability and physical integrity to the implant
- A hard segment-for the thermoplastic elastomer-- an isocyanate, polyester or polyamide-- which is non cytotoxic within the intended therapeutic usage period of the implant.
- Solubility of the active agent in the amorphous component (soft chemical segment) of the reservoir copolymer or polymer is also important to controlled drug delivery rate over the functional life of the implant.

A skilled person in the art can select the appropriate polymer or polymer blend and additives (e.g. excipients) to achieve the desired therapeutic blood level of a given active agent.

For a different active drug or combination of drugs, or different therapeutic indications in human or animal subjects, the skilled person will specify a different set of release kinetics. It is possible to select from a series of polymeric resins or resin blends to achieve the desired kinetics and optimum therapeutic blood levels for specific human or animal indications for hydromorphone and other selected drugs or combinations of drugs.

### Thermoplastic Polyurethanes (TPUs)

TECHOFLEX® Medical Grade Thermoplastic Polyurethanes (Grades EG- 80A, EG-85A, EG- 93A and EG-60D) comprise a group of aliphatic, polyether based resins that have established credentials for implants including having passed the following standard screening tests: MEM Elution, Hemolysis, USP Class VI, 30 Day Implant, and Ames Mutagenicity.

These urethane resins have been evaluated in several medical device applications that involve the requirement for high permeability to moisture vapor. They are highly amorphous compounds which allow them to be used for drug delivery systems where high loading and flux rate are required.

TECHOFLEX® EG-80A and Tecoflex EG-85A are both made from the same diisocyanate (HMDI) and the same 2000 molecular weight PTMEG polyol but the ratios of polyol to diisocyanate (hard segment to soft segment) are different. The lower modulus, lower Tg version - Tecoflex EG-80A - is more amorphous and less crystalline in its morphology resulting in a higher flux drug delivery formulation. Tecoflex EG-60D is based on the same HMDI diisocyanate but a 1000 molecular weight PTMEG polyol, resulting in a different morphology, crystallinity and drug flux.

A series of specific formulations can be made using various combinations of the above Tecoflex resins.

Other thermoplastic polyurethanes, including Tecoflex EG-85A, EG-93A or EG-60D, can be used alone or blended together with hydromorphone HCI or other drugs to form the feedstock for the internal polymer matrix, or without the drug to form the drug impermeable coating. Tecoflex EG-80A is a medical-grade, aliphatic, polyether-based thermoplastic polyurethane elastomer with a durometer value of 72A. Tecoflex EG-85A is a medical-grade, aliphatic, polyether-based thermoplastic polyurethane elastomer with a durometer value of 77A. CARBOTHANE® PC-3575A TPU is a medical-grade, aliphatic, polycarbonate-based thermoplastic polyurethane elastomer with a durometer value of 73A. CARBOTHANE® PC-3585A is a medical-grade, aliphatic, polycarbonate-based thermoplastic polyurethane elastomer with a durometer value of 84A.

Certain thermoplastic polyurethanes have been specifically developed for long term (90 days and beyond) human implants including extended release drug delivery systems. These polymers, either used singly or as blends, are advantageous reservoir components and include but are not limited to the following:
Elasthane thermoplastic polyether polyurethane resins are formed by the reaction of polytetramethyleneoxide and an aromatic diisocyanate. They may be custom synthesized with selected functional chemical end groups which impact the uniform delivery rate of the device. An important feature which can be built into the TPU is increased hydrophilic properties which result in more efficient access of body fluids to the aqueous soluble drug substance e.g. hydromorphone HCL, uniformly dispersed throughout the TPU matrix.

This functional enhancement in hydrophilicity is an important formulation tool which can be used to correct and improve the tendency of hot melt systems to reduce availability of active drug components by surrounding and encasing particles of the active drug product (API) in such a way as to restrict access to body fluids. Increasing hydrophilic properties of the TPU improves transport of body fluids into and through the surface of the central channel and down into throughout the entire polymeric matrix.

BIONATE® thermoplastic polycarbonate polyurethanes are a family of thermoplastic elastomers formed as a reaction product of a hydroxyl terminated polycarbonate, an aromatic diisocyanate and a low molecular weight glycol to form the soft segment. This family of products is well suited for long term (90 days or more) versions of the drug delivery implant.

BioSPAN® segmented polyether polyurethanes are a third category of TPU resins which are particularly useful for manufacturing the implant using a solution based processes. This material is one of the most extensively tested human implant grade polyurethane and has been specifically developed for solution systems.

A composition for the core matrix is an aliphatic, polyether-based, thermoplastic polyurethane compatible with hydromorphone HCI or other opioids. A specific grade of aliphatic, polyether-based, thermoplastic polyurethane -- identified as Tecoflex EG80A possesses sufficient solubility in the processing solvent (methylene chloride) to allow for efficient production of the core matrix.

See WO 2010/120389 for a discussion of thermoplastic resin blends and functional excipients and plasticizers.

### Silicone Coatings

The coatings of the invention are impermeable to the therapeutic agent such as an analgesic (e.g. opioid). The coating, the purpose of which is to restrict the release of drug to the surface area of uncoated polymer in the central channel, allows uniform controlled flux with no burst effect. The coating is a significant factor in preventing possible leakage of the active opioid (or other drug) and a potentially uncontrolled and lethal burst effect while the implant is in use. In the subject invention, the coating includes a silicone polymer.

Examples of silicone materials useful for coating the subcutaneous delivery system of the invention are disclosed in US Patents 3,035,016, 3,077,465, 3,274,145, 3,636,134, 3,647, 917 and 4,115,356.

The materials used to coat are medical-grade silicone adhesives and dispersions. It was discovered that silicone adhesives and dispersions can be used by themselves as coating materials or as part of the coating. The silicone adhesives are either neat adhesives (e.g. Applied Silicone Corporation, Product No. 40064 and 40076) or dispersions of silicone prepolymers in a pharmaceutically acceptable solvent (e.g. Applied Silicone Corporation, Product No. 40000, 40021, and 40130, or their equivalents from other vendors).

When used, these prepolymers cross-link to form an extended polymer network that cannot be redissolved. Depending on the specific composition, the silicone adhesive or dispersions can cross-link at room temperature. This cross-linking process is fundamentally different than solvent-processing of solutions containing polymers that do not cross-link. The use of cross-linking polymers such as the silicones identified is typically not considered for use by drug product formulators. Formulators typically select thermoplastic polymers that can be solvent-processed in pharmaceutically acceptable processing solvents or to polymer constructs that have been previously processed such as extruded films.

### Silicone Dispersion

Specifically related to the finished product characteristics, silicone dispersion is prepared as a dispersion of polydimethylsiloxane prepolymers of limited degree of polymerization (DP) in hydrophobic organic solvent. For the silicone dispersion samples used in the subcutaneous drug delivery system described in this disclosure, the organic solvent was xylene (e.g. Applied Silicone Corporation Product No. 40000 and 40021) or perchloroethylene/butyl acetate (e.g. Applied Silicone Corporation Product No. 40130).

Total solids content for these products are between 18 and 40%, depending on the specific grade. As a result of using the silicone dispersion to coat the core matrix, residual organic solvent can remain in the core matrix.

### Silicone Adhesives

As an alternative to silicone dispersions, silicone adhesives are not provided in organic solvent. Silicone adhesives are often made of high-molecular-weight poly-dimethyl siloxane (silicone gum) and silicate resin, which consists of (CH₃)₃SiO and SiO₂ units. The ratio of silicone gum to silicate resin is 100/50 to 100/150 by weight.

Total solids content in the adhesives are 100% polydimethylsiloxane prepolymers of limited degree of polymerization (DP). When used in the construction of subcutaneous drug delivery system, silicone adhesive does not contribute any residual solvent to the core matrix and does not affect the inherent diffusional resistance of the core matrix. Support for this was subsequently observed from results of in vitro dissolution assays with subcutaneous drug delivery system samples prepared with silicone adhesive that were consistent to that predicted to be achieved from subcutaneous drug delivery formulations of similar core matrix dimensions and prepared with coating systems containing hydrophilic organic solvents.

To one skilled in the art, silicone adhesives are to be used as construction aids in the assembly of integral components. Due to their adhesive nature, they are inherently difficult to use for other than the intended purpose. However, a construction technique has been developed in which isopropanol is used as a wetting agent for the blade used to consistently form and smooth the silicone adhesive into the coating layers used to construct the subcutaneous drug delivery system samples (see Examples).

Advantages of using cross-linked silicone as the coating material are, but not limited, to the following:
- Cross-linked silicones are inherently impermeable. Current dissolution results for cross-linked silicone coated sample without center holes have shown zero hydromorphone release over 42 days.
- The production technique for manufacturing the subcutaneous drug delivery system under cGMP is simplified. Research indicates that one layer of cross-linked silicone achieves impermeability to hydromorphone. The drug delivery system manufacturing involves production of cores by the solvent-processing technique followed by encapsulating the cores within the silicone coating by web-coating techniques, cross-linking the silicone coating by curing at room temperature, and, finally, punching out completed drug delivery systems with center holes.
- Greater assurance of defect-free production. The silicone coating forms a continuous envelope surrounding the core matrix with no potential for edge defects that may occur with other construction techniques.
- The silicone adhesives and dispersions have an extensive use history in the medical device industry, established toxicology profile, and proven regulatory acceptance.
- The resulting cross-linked silicone coatings are inherent tough and flexible. Because it is cross-linked, the silicone coatings are also potentially less sensitive to heat unlike coating materials applied by a solvent-processing technique that do not involve cross-linking.

Silicone adhesives and dispersions used in the practice of this invention can be cross-linked by either platinum-catalysis (such as Applied Silicone Corporation, Product No. 40000 and 40130 or acetoxy-catalysis such as Applied Silicone Corporation, Product No. 40021, 40064, and 40076). In addition, the silicone adhesive or dispersion used in the practice of this discovery can be heat-cured at elevated temperatures or cured at room temperature (i.e. room-temperature vulcanization or RTV), depending on the specific composition of the silicone adhesive or dispersion.

Alternatives to silicone include use of pharmaceutically acceptable, cross-linking adhesives including cyanoacrylate adhesives, acrylate-based resin systems, one- or two-component epoxy resin systems, and light or UV-curable acrylic or epoxy resin systems.

### Biodegradable Polymers

In a further embodiment of the disclosure, a subcutaneous drug delivery system is provided comprising a biocompatible, biodegradable thermoplastic polymer matrix, a therapeutic agent embedded homogeneously in said matrix, and a biocompatible, biodegradable drug impermeable polymer coating said matrix, wherein said delivery system has geometry such that there is an external coated wall and an internal uncoated wall (or channel) forming an opening for release of said therapeutic agent, and the distance between the uncoated wall and the coated wall opposite the uncoated wall is substantially constant throughout the delivery system. Methods of producing and using such delivery systems are also provided.

In a further embodiment of the disclosure, a subcutaneous drug delivery system is provided comprising a biocompatible, biodegradable thermoplastic polymer matrix, a therapeutic agent embedded homogeneously in said matrix, and a biocompatible drug impermeable polymer coating said matrix, wherein said delivery system has geometry such that there is an external coated wall and an internal uncoated wall (or channel) forming an opening for release of said therapeutic agent, and the distance between the uncoated wall and the coated wall opposite the uncoated wall is substantially constant throughout the delivery system. The disclosure also relates to the methods of producing and using such delivery systems.

A further embodiment of the disclosure relates to a subcutaneous drug delivery system comprising a biocompatible thermoplastic polymer matrix, a therapeutic agent embedded homogeneously in said matrix, and a biocompatible, biodegradable drug impermeable polymer coating said matrix, wherein said delivery system has geometry such that there is an external coated wall and an internal uncoated wall (or channel) forming an opening for release of said therapeutic agent, and the distance between the uncoated wall and the coated wall opposite the uncoated wall is substantially constant throughout the delivery system. The disclosure also relates to the methods of producing and using such delivery systems.

A skilled person in the art can select the appropriate biodegradable polymer or polymer blend and additives (e.g. excipients) to achieve the desired therapeutic blood level of a given active agent.

For a different active drug or combination of drugs, or different therapeutic indications in human or animal subjects, the skilled person will specify a different set of release kinetics. It is possible to select from a series of polymeric resins or resin blends to achieve the desired kinetics and optimum therapeutic blood levels for specific human or animal indications for hydromorphone and other selected drugs or combinations of drugs.

Advantages of using biocompatible, biodegradable polymers as the core matrix and/or coating material are, but not limited, to the following:
- By selecting biodegradable polymers with *in vivo* degradation rates substantially slower than the target release rate of therapeutic agent, the degradation of the biodegradable polymer, whether employed as a component of the core matrix or as the impermeable polymer coating, would have no effect on the release rate of the therapeutic agent.
- Subcutaneous drug delivery systems prepared with biodegradable polymer, whether employed as a component of the core matrix or as the impermeable polymer coating and selected to have *in vivo* degradation rates substantially slower than the target release rate of therapeutic agent, would remain intact and retrievable during the intended delivery duration to facilitate removal if needed during the course of therapy.
- After the intended delivery duration, subcutaneous drug delivery systems prepared with biodegradable polymer, whether employed as a component of the core matrix or as the impermeable polymer coating and selected to have *in vivo* degradation rates substantially slower than the target release rate of therapeutic agent, would degrade wholly or in part.
- When degraded wholly *in vivo,* removal of depleted subcutaneous drug delivery systems would not be needed after administration.
- When degraded in part *in vivo* whether as a component of the core matrix or as the impermeable polymer coating, release of remaining residual therapeutic agent would occur. In instances of repeated administration of subcutaneous drug delivery systems, *in vivo* degradation the biodegradable polymer as either a component of the core matrix or as the impermeable polymer coating would prevent accumulation of multiple depleted subcutaneous drug delivery systems releasing residual therapeutic agent which over time would result in blood levels of therapeutic agent not completely defined by the target release rate of newly administered subcutaneous drug delivery system.
- Biodegradable polymers have an extensive use history in the medical device industry, established toxicology profile, and proven regulatory acceptance.

### Methods of Production

Manufacturing processes capable of large scale production of the drug/polymer formulations described herein can comprise the following processes for production of the drug reservoir matrix and subsequent coating or layering of a diffusional resistance-impermeable coating surrounding the drug reservoir matrix. Included in the manufacturing processes is also the generation of the drug releasing hole through the center of the drug reservoir matrix. The surface area in the drug reservoir matrix resulting from the generation of the drug release hole is not coated or layered with a diffusional resistance coating. Generation of the drug release hole can be accomplished before or after coating or layering the diffusional resistance coating surrounding the drug reservoir matrix.

### Drug reservoir matrix:

- Solution (Solvent) casting of the components of the drug reservoir matrix into molds of specified dimensions.
- Solution casting of the components of the drug reservoir matrix and web-coating a film.

### Diffusional Resistance (Impermeable) Coating:

- Web-coating the diffusional resistance coating with subsequent application of individual or multiple drug reservoir matrix(ces)
- Dip coating of individual or multiple drug reservoir matrix(ces)
- Spray application of coating to individual or multiple drug reservoir matrix(ces)
- Hot-melt application of coating to individual or multiple drug reservoir matrix(ces)
- Powder coat application of coating to individual or multiple drug reservoir matrix(ces) and annealing.

### Center Hole Generation:

- Use of mechanical drill
- Use of die punch
- Use of a laser drill
- Preformed casting mold

### Solution Based Polymeric Drug Delivery Device (Solution or Solvent Casting)

The three dimensional composition and configuration of the drug delivery device can also be accomplished by pouring or injecting the solvent based formulation into a mold or multi-cavity mold. This approach eliminates most of the thermal issues involved with multiple pass coating and drying. Using this approach, the solution based formulations, having been filled into the mold, can be allowed to dry slowly at reduced or ambient temperatures, thereby reducing or eliminating high temperature related decomposition of polymer or active drug component.

More specifically, a polyurethane, copolyester or polyether block amide is mixed with a polar solvent (such as DMF or methylene chloride) to form a polymer solution. The active agent, e.g. hydromorphone, is then added to the solution. The solution or suspension is poured or introduced into a mold which forms the three dimensional shape of the implant. The implant is dried in such a way as to eliminate the solvent. Alternatively, the solution is dried as a flat sheet and then the sheet is die cut to form the desired shape, e.g. a circular disc. The implant is then coated.

### Coating of the Core Matrix

The coating process can be batch or continuous depending on the volume needed for commercial production.

An advantageous continuous process is described below.
1. Drug-containing cores are prepared by casting and drying a processing suspension comparing drug substance and core polymer matrix. Continuous web-coating process can be used to prepare and dry a core film that is cut into individual implant cores.
2. Uncured polydimethyl siloxane is applied to a suitable manufacturing substrate in a continuous web-coating operation using a knife-over-roll or equivalence station. The manufacturing substrate is identified as such to have sufficient non-stick properties to prevent finished cured polydimethyl siloxane film from adhering and release the finished film for additional processing. Coating thickness is controlled by the wet-gap defined between the manufacturing substrate and the knife blade.
3. Drug-containing cores are placed onto the uncured polydimethyl siloxane film. Placement can be achieved using a mechanical pick-and-place station on the continuous web-coating operation to achieve registration onto the uncured polydimethyl siloxane film.
4. A second uncured polydimethyl siloxane film is applied to a suitable manufacturing substrate in a continuous web-coating operation using a second knife-over-roll or equivalence station to completely enclose the core.
5. The completed film/core/film is cured at a suitable temperature - room temperature for RTV (room temperature vulcanizable) silicone adhesive grades. If non-RTV silicone adhesive grades are used, elevated temperatures will be needed for cure.
6. Once cured, the individual finished implants are cut for the film/core/film and packaged.

### Hot Melt Application of Coating and Hybrid Construction of Implants

In one embodiment, hot-melt processed (extruded) films are used as the coat for the drug delivery system. To make this approach successful in the construction of the subcutaneous drug delivery system, a method is needed to establish a strong bond between the extruded film and the core matrix. By the use of a silicone adhesive tie-layer, tight bonding of the film to the core matrix can be achieved. Film materials useful in the invention have the attributes of impermeability to hydromorphone, pharmaceutically acceptable, possessing flexibility and toughness, and capable of achieving regulatory approval. High durometer TPU's possess these attributes and were used to prepare samples by the hybrid construction technique for dissolution testing. See US application 2010/0303883 and WO 2010/120389. In the preparation of these samples, extruded film samples were obtained and a silicone adhesive tie-layer used that was essentially a polymeric dip-coating solution to prepare subcutaneous drug delivery system samples. Film samples investigated were neat TPU resins and TPU resin mixtures containing nanocomposite material proprietary to the vendor supplying the extruded films.

Co-extrusion enables i) multi-layer external polymer construction, insuring against leaks due to pinholes, ii) the manufacture of a multi-layer composite external polymer wherein a specific polymeric drug barrier is included in the structure- insuring against uncontrolled diffusion of active resulting in a burst effect during use, and iii) the manufacture of a multi-layer composite external polymer including a specifically selected silicone adhesive tie coat to secure and optimize physical and structural integrity of the implant by enhancing the bond between components.

### Radio-Opaque Markers

Radio-opaque pigments; e.g., TiO2, or barium sulfate, can be conveniently added to either or both exterior or interior polymers enabling the implant to be easily located by X-ray in the event removal is required or useful. Other imbedded markers have the potential of providing important information about the implant once in place in the patient including dose in ug/hr, expected duration of release of the active analgesic (hydromorphone HCl) and date of implantation. Such information can be linked to a database available to physicians.

### Exemplary Uses of the Implants

The delivery systems disclosed herein are useful for delivery of therapeutics for extended periods of time, e.g. 2 days to six months, or 1 or 2 weeks to 6 months.

### Delivery of Opioids

Certain other embodiments also include delivery systems for use in treating pain, e.g. cancer pain, by subcutaneous administration of a delivery system containing an analgesic, advantageously an opioid such as hydromorphone.

Other opioids useful in the subject invention include morphine analogs, morphinans, benzomorphans, and 4-phenylpiperidines, as well as open chain analgesics, endorphins, encephalins, and ergot alkaloids.

Advantageous compounds, because of their potency, are etorphine and dihydroetorphine which are 1,000 to 3,000 times as active as morphine in producing tolerance to pain (analgesia). 6-methylene dihydromorphine is in this category, also, and is 80 times as active as morphine. Buprenorphine (20-40 x morphine) and hydromorphone (6-7 x as potent as morphine) also belong to this class of compounds. These five compounds, and many more, are morphine analogs.

The category of morphinans includes levorphanol (5 x morphine). A compound from this group is 30 times more potent than levorphan and 160 x morphine. Fentanyl, a compound that does not follow all the rules for 4-phenylpiperidines, is about 100 times as potent as morphine.

The benzomorphan class includes Win 44, 441-3, bremazocine and MR 2266 (see Richards et al., Amer. Soc. for Pharmacology and Experimental Therapeutics, Vol. 233, Issue 2, pp. 425-432, 1985). Some of these compounds are 4-30 times as active as morphine.

### Delivery of Other Active Agents where a Burst is Dangerous

Advantages of the subject delivery system are that it provides systemic delivery, burst free, constant release, long duration. Thus, the system is advantageous for situations where burst might be dangerous - examples are the delivery of anti-hypertensives and antiarrhythmics.

### Delivery of Active Agents where Drug is Wasted in Burst

Another situation is where drug is wasted in burst. Examples are: Infectious disease-antibiotics, antivirals, antimalarials, anti-TB drugs, hormones or hormonal blockers, androgens, estrogens, thyroid drugs, tamoxifen, antiseizure drugs, psychiatric drugs, anti-cancer drugs, antiangiogenics, and vaccines.

### Delivery of Active Agents where Compliance is Important

The implant is useful in the delivery of active agents where compliance is important such as in the treatment of opioid addiction by administration of methadone or hydromorphone.

### Veterinary Applications

The implants of the subject invention can also be used as noted above for corresponding veterinary applications e.g. for use in delivering active agents such analgesics, such as hydromorphone or etorphine to dogs or cats.

The following Examples are illustrative, but not limiting of the compositions and methods of the present invention. Other suitable modifications and adaptations of a variety of conditions and parameters normally encountered which are obvious to those skilled in the art are within the scope of this invention.

### EXAMPLES

### Example 1 - Hydromorphone Release Rate Assay

Hydromorphone release rate from either uncoated or coated drug reservoir matrix were determined using the following analytical method.

Release media was a pH 7.4 sodium phosphate buffer prepared by dissolve 2.62 g of monobasic sodium phosphate and 11.50 g of anhydrous dibasic sodium phosphate into 1 L of DI water. The preparation was mixed well until added components were dissolved.

Uncoated or coated drug reservoir matrices were analyzed for hydromorphone release rate by placing one matrix (after weighing) in to a 25-mL screw cap centrifuge tube. Add 10 mL of 0.1 M sodium phosphate, pH 7.4, release media to the tube. Cap and wrap a piece of flexible laboratory film such as Parafilm® around centrifuge tube cap. Place all centrifuge tubes in a water bath maintained at ∼37°C and start timer.

After desired amount of time, remove the release media from the centrifuge tube using a syringe and canula and place the release media into a clean test tube. Add fresh 10 mL of release media to the sample test tubes and place back in water bath if necessary to continue release rate assay.

Hydromorphone standards were prepared to a concentration of ∼0.5 mg/mL. Accurately weigh about 25 mg of hydromorphone HCl and transfer to a 50-mL volumetric flask. Rinse and dilute to volume with pH 7.4 release media. This solution is good for about 7 days on bench top at ambient conditions.

Release media samples were analyzed by spectrophotometry using a spectrophotometer set at a wavelength of 280 nm and using a 0.2-cm cell path length. The spectrophotometer was initialized with the pH 7.4 phosphate buffer. The hydromorphone standard solution was analyzed 5 times and the absorbance was measured. Calculate the relative standard deviation in the absorbance measurement and verify that the value is less than 2.0% RSD before proceeding with analyzing the release media samples. If necessary, the release media sample solutions can be diluted down with pH 7.4 phosphate buffer if the initial absorbance is too high. Bracket analysis of the release media samples with analyses of hydromorphone standards with no more than 12 sample readings between standards reading and complete the assay with a hydromorphone standard reading. Verify that the %RSD is remains less than 2.0%.

### Example 2 - Thermoplastic Polyurethane Matrix

Thermoplastic polymers were investigated as the drug reservoir matrix. Hydromorphone HCl (to produce a 70% wt/wt hydromorphone HCl to aliphatic, polyether-based, thermoplastic polyurethane (Tecoflex® EG80A)) was suspended in approximately 22% w/w Tecoflex EG80A/methylene chloride solution. Specifically, 4.90 g of hydromorphone HCI was suspended in a solution prepared by dissolving 2.10 g of Tecoflex EG80A in 25.0 g of methylene chloride. Sufficient time for complete dissolution of Tecoflex EG80A should be allowed before adding hydromorphone HCl. In this Example, the Tecoflex EG80A was allowed to dissolve in methylene chloride for approximately 3 days without adverse effect on the suspension casting process. The suspension was mixed for at least 4 hours and then cast into 100-mm glass Petri dish at room temperature. The cast film was allowed to air dry at room temperature without applied vacuum. After less than 24 hours, the resulting cast was a dry, flexible, easily removed from dish. The cast film was cut to produce 9/16" drug reservoir matrices with weights of between 124 and 203 mg with a mean of 151 mg and with thicknesses of between 0.68 and 1.29 mm with a mean of 0.895 mm.

### Example 3 - Thermoplastic Polyurethane Matrix

Thermoplastic polymers were investigated as the drug reservoir matrix. Hydromorphone HCl to produce a 70% wt/wt hydromorphone HCl (to aliphatic, polyether-based, thermoplastic polyurethane (Tecoflex® EG80A)) was suspended in approximately 22% w/w Tecoflex EG80A/methylene chloride solution. Specifically, 12.95 g of hydromorphone HCl was suspended in a solution prepared by dissolving 5.55 g of Tecoflex EG80A in 66.0 g of methylene chloride. Sufficient time for complete dissolution of Tecoflex EG80A should be allowed before adding hydromorphone HCl. In this Example, the Tecoflex EG80A was allowed to dissolve in methylene chloride for approximately 4 days without adverse effect on the suspension casting process. The suspension was mixed for approximately 2 - 4 hours and then cast into 150-mm glass Petri dish at room temperature. The cast film was allowed to air dry at room temperature without applied vacuum. After less than 24 hours, the resulting cast was a dry, flexible, easily removed from dish. The cast film was cut to produce ½" drug reservoir matrices with weights of between 126 and 191 mg with a mean of 151 mg and with thicknesses of between 0.92 and 1.32 mm with a mean of 1.13 mm.

### Example 4 - Thermoplastic Polyurethane Matrix with Silicone Dispersion Coating

The core matrix containing hydromorphone HCl was produced as in Example 2 above.

To form the bottom coating layer, uncured silicone dispersion with 5 wt% BaSO₄ in xylene (Applied Silicone Corporation, custom manufacture based on Product No. 40021) was web-coated onto a Teflon substrate with glass framework of known thickness to form a coating gap. Silicone dispersion was filmed onto the Teflon substrate using a stainless steel straight-edge to smooth the silicone dispersion into an uncured film. The film was allowed to cure for a minimum of 1 hour before further processing. A small amount of uncured silicone dispersion was placed on one surface of the drug reservoir matrix with the appropriate target drug loading and, the matrix was manually placed on the first layer of uncured silicone dispersion film with silicone dispersion augmented side down and pressed down to insure complete contact between the silicone film and the drug reservoir matrix. The function of additional silicone dispersion placed on the drug reservoir matrix was to facilitate adhesion to the bottom silicone dispersion film.

Addition layer of glass framework was added onto the existing framework to increase the coating gap to facilitate the formation of the top coating layer of known thickness to form a coating gap. To form the top coating layer, uncured silicone dispersion with 5 wt% BaSO₄ in xylene was web-coated onto the bottom silicone dispersion layer onto which the drug reservoir matrices have been adhered. Silicone dispersion was filmed onto the bottom silicone dispersion layer using a stainless steel straight-edge to smooth the silicone dispersion. The bottom and top silicone dispersion layers were allowed to cure at room temperature for a minimum of 24 hours before additional processing after which the samples were placed under partial vacuum for a minimum of 4 hours to facilitate removal of residual xylene. Total mean thickness of samples was 2.73 mm.

Once cured, the samples were manually cut from the web sheet using a 5/8" arch punch in such a manner as to center the drug reservoir matrix inside the arch punch and insure when cut a uniform thickness of cured silicone dispersion around the perimeter of the drug reservoir matrix. Finally, a center hole was manually cut through the sample using 19G stainless steel tubing using a punch technique in which a high-speed rotary tool is not used to facilitate cutting the center hole.

The coated drug reservoir matrices that attained target weight were assayed for hydromorphone release using the analytical method described in Example 1. The results are shown in Figure 1.

### Example 5 - Thermoplastic Polyurethane Matrix with Silicone Adhesive Coating

The core matrix containing hydromorphone HCI was produced as in Example 3 above.

To form the bottom coating layer, uncured silicone adhesive (Applied Silicone Corporation, Product No. 40076) was web-coated onto a Teflon substrate with glass framework of known thickness (∼0.5 mm) to form a coating gap. Silicone adhesive was filmed onto the Teflon substrate using a stainless steel straight-edge to smooth the silicone adhesive into an uncured film thickness of ∼0.5 mm. A small amount of uncured silicone adhesive was placed on one surface of the drug reservoir matrix with the appropriate target drug loading. Subsequently, the matrix was manually placed on the first layer of uncured silicone adhesive film with silicone adhesive augmented side down and pressed down to insure complete contact between the silicone film and the drug reservoir matrix. The function of additional silicone adhesive placed on the drug reservoir matrix was to facilitate adhesion to the silicone adhesive film.

Addition layer of glass framework was added onto the existing framework to increase the coating gap to facilitate the formation of the top coating layer of known thickness (total gap ∼2 mm) to form a coating gap. To form the top coating layer, uncured silicone adhesive (Applied Silicone Corporation, Product No. 40076) was web-coated onto the bottom silicone adhesive layer onto which the drug reservoir matrices have been adhered. Silicone adhesive was filmed onto the bottom silicone adhesive layer using a stainless steel straight-edge to smooth the silicone adhesive into an uncured film ∼2 mm in total sample thickness. The bottom and top silicone adhesive layers were allowed to cure at room temperature for a minimum of 24 hours before additional processing. Total mean thickness of samples was 1.95 mm.

Once cured, the samples were manually cut from the web sheet using a 9/16" arch punch in such a manner as to center the drug reservoir matrix inside the arch punch and insure when cut a uniform thickness of cured silicone adhesive around the perimeter of the drug reservoir matrix. Finally, a center hole was manually cut through the sample using 19G stainless steel tubing. To facilitate cutting the center hole, the tubing was attached to a high-speed rotary tool and spun at high-speed.

The coated drug reservoir matrices that attained target weight were assayed for hydromorphone release using the analytical method described in Example 1. The results are shown in Figure 2.

### Example 6 - Rabbit Implant Study

A GLP grade study of the safety and performance of the hydromorphone disk implant ("HDI") was conducted at Covance Laboratories, Inc. In this study 10 male and 10 female New Zealand white rabbits each had one HDI implanted just under the skin on their backs (dorsal side). For both male and female rabbits five (5) animals were randomized to a "toxicity" group and five (5) were randomized to a "toxicokinetics" group. All animals were examined at cageside on a twice daily basis, food intake was recorded daily, and weight gain data were collected on a twice weekly basis. The animals in the "toxicity" group were also examined clinically on a regular basis and subjected to macroscopic and microscopic pathology examinations at terminal sacrifice or at any interim event of mortality. The animals in the "toxicokinetics" group had blood samples collected on a protocol schedule and serum hydromorphone concentration was determined in these samples. "Toxicokinetic" animals did not receive regular clinical examinations or detailed macroscopic or microscopic pathology examinations. Many animals (11 out of the 20 implanted) opened up or otherwise damaged their implant sites to an extent such that surgical repair was necessary. The following table provides a listing of these animals (note that #F27849 required repair twice):

**Incision Site Repair**

| Animal Number | Group | Sex | Dosing Phase Day of Incision Site Repair | Repair Procedure |
|---|---|---|---|---|
| F27849*^ | 1 | Male | 1 | a |
| F27851 | 2 | Male | 1 | a |
| F27855 | 2 | Male | 1 | a |
| F27860* | 1 | Female | 1 | a |
| F27865 | 1 | Female | 1 | a |
| F27849*^ | 1 | Male | 2 | a |
| F27861 | 1 | Female | 3 | b |
| F27863 | 1 | Female | 3 | b |
| F27863 | 1 | Female | 4 | c |
| F27856* | 1 | Female | 4 | c |
| F27864 | 1 | Female | 9 | c |
| F27857* | 1 | Female | 14 | c |

| | | | | |
|---|---|---|---|---|
| a repair following local anesthesia (lidocaine) b repair following local anesthesia (buproicaine) c repair following general anesthesia * Animal in the toxicokinetics group. ^ Required repair on two occasions. | | | | |

The above tabulation is only for those animals who damaged their implant sites sufficiently as to require a post-implant surgical repair. Scratching or otherwise pawing at surgical wound sites is a common phenomenon in rabbits and many other animal species. It can be noted that if approximately 50% of these animals required surgical repair of their implant sites and that scratching or pawing at wound sites is a common phenomenon in rabbits then it can be assured to a reasonable degree of scientific certainty that all the animals in this study were scratching or pawing at their implant sites. It can further be noted that the greatest proportion (9 of 11) of animals requiring surgical repair of their implant sites occurred within the first 4 days post-implant and 5 of 11 such repairs were required at one (1) day post-implant. This suggests that scratching and/or pawing at the implant sites was likely most aggressive during the first week post-implant and then likely tended to a reduced activity as the animals became more accustomed to the implant placements.

The implants were not secured within their implant pockets (by design as it is not intended in the human application to secure the implant beyond placing it into an implant pocket) and even absent an overt opening of the implant wound (as occurred in 11 of 20 animals) scratching and/or pawing at the implant site would be expected to move the implant within the pocket and, given that the HDI is a hard plastic disk, such movement would be expected to induce trauma and associated bleeding within the pocket itself. Therefore, either by bleeding occurring at the time of overt opening of the implant wound (in 11 of 20 animals) or internal bleeding within the implant pocket (as likely occurred in all animals to some extent) the implant channel would be expected to have blood enter it with subsequent deposition of thrombus within the channel. This is in fact what occurred as all explanted HDI's that were available for examination showed remnants of blood and thrombus within the central channel.

Despite the above noted issues with animal husbandry and protection of the implant sites, the implanted HDI's in the "toxicokinetic" animals functioned to release hydromorphone into circulation in a sustained release mode of delivery. This is seen in FIGS. 3 and 4, which are graphs of hydromorphone serum levels as a function of days post-implantation for male and female rabbits, respectively.

In this study the mean serum hydromorphone rises to approximately 15,000 ng/mL and remains at that level for the first several days post-implant. It then begins to decline slowly, falling to approximately 10,000 ng/mL in the period 10 - 15 days post-implant and to approximately 5,000 ng/mL by day 30 post-implant. This decline in mean serum hydromorphone starts sooner than was anticipated on the basis of *in vitro* release kinetics from the HDI used. This sooner than anticipated decline in serum hydromorphone in this study can be attributed to clogging of the central channel with thrombus which will progressively reduce the release rate of the hydromorphone from the HDI. The *in vitro* hydromorphone release rate from the HDI's that were used is essentially zero order (i.e.: linear over time) until the HDI's hydromorphone content is depleted to an extent that there is insufficient concentration gradient to drive diffusion release. The expected performance of the HDI *in vivo* was for decline to begin at some time in the 25 - 30 day window and that the hydromorphone load would be essentially depleted at or around 30 days post-implant. In fact approximately 15% of the hydromorphone load remained in the implants at day 30 and this indicates that the *in vivo* release rate was less than predicted from the *in vitro* data. This observation is consistent with the data shown in Figures C1 and C2 and, also, with the concept that thrombus clogging of the central channel resulted in a progressive diminution of the *in vivo* release rate which in turn resulted in the slow decline observed in the rabbit implant study.¹
¹ It is important to note that the data do not support a complete cessation of release as in that case the decline in serum hydromorphone would have been driven by the kinetics of elimination of hydromorphone from circulation and hydromorphone levels would have declined to baseline within a day. The slow decline seen here is only consistent with a gradually decreasing rate of hydromorphone release from the HDI.

With regard to safety, there were no suggestions of systemic toxicological effects due to the HDI or its contained hydromorphone and, aside from *sequelae* of the above-noted scratching and/or pawing at the implant sites, there were no local implant site effects aside from normal, expected post-surgical observations.

The results of this study demonstrate that:
(a) The HDI functions effectively for sustained release of hydromorphone after subcutaneous implantation;
(b) There are no significant sex differences in the release performance; and,
(c) The HDI is safe for such implantation and in a human would be well tolerated.

It will be readily apparent to those skilled in the art that numerous modifications and additions may be made to the present invention, the disclosed device, and the related system without departing from the invention disclosed.

## Claims

1. A subcutaneous delivery system comprising:
(i) a biocompatible thermoplastic elastomer matrix,
(ii) a therapeutic agent dispersed homogeneously in said matrix, and
(iii) a biocompatible therapeutic agent impermeable cross-linked silicone polymer coating said matrix,
wherein said delivery system has a geometry such that there is an external coated wall and an internal uncoated wall forming an opening for release of said therapeutic agent, and the distance between the uncoated wall and the coated wall opposite the uncoated wall is substantially constant throughout the delivery system.

2. A subcutaneous delivery system as in claim 1, wherein said delivery system is cylindrical in shape.

3. A subcutaneous delivery system as in claim 1, wherein said matrix is a polyurethane matrix; preferably wherein said urethane matrix has an isocyanate as a hard segment, and a PEG, PPG or PTMEG glycol soft segment.

4. A subcutaneous delivery system as in claim 1, wherein said matrix is a copolyester matrix; preferably wherein said copolyester matrix has a polyester as a hard segment, and a PEG, PPG or PTMEG glycol soft segment.

5. A subcutaneous delivery system as in claim 1, wherein said matrix is a polyether block amide matrix; preferably wherein said polyether block amide matrix has a polyamide as a hard segment, and a PEG, PPG or PTMEG soft segment.

6. A subcutaneous delivery system as in claim 3, 4 or 5, wherein the hard segment is 20-70% by weight of the matrix polymer with the remainder the soft segment; or wherein approximately 50% of the therapeutic agent is in solution with the soft segment of the matrix polymer while the remaining portion of the therapeutic agent is dispersed in the matrix and not in solution.

7. A subcutaneous delivery system as in claim 1, wherein said matrix and coating are non-biodegradable; or wherein the silicone is a crosslinked polyorganosiloxane; or wherein the silicone is crosslinked polydimethylsiloxane.

8. A subcutaneous delivery system as in claim 1, wherein said therapeutic agent is an opioid, such as Buprenorphine; or wherein said therapeutic agent is selected from the group consisting of hydromorphone, etorphine and dihydroetorphine; preferably wherein said therapeutic agent is hydromorphone.

9. A subcutaneous delivery system as in claim 1, wherein said matrix is a polyurethane, such as a polyether based polyurethane, and said coating is crosslinked polydimethylsiloxane.

10. A subcutaneous delivery system as in claim 1, wherein said silicone polymer coating is an adhesive tie coat between said polymer matrix and a second coat comprising a second biocompatible therapeutic elastomer matrix.

11. A subcutaneous delivery system as in claim 10, wherein said second coat is a copolyester, a polyether block amide, or a thermoplastic polyurethane;
or wherein said second coat contains a second therapeutic agent;
or wherein each coating is 24-48 microns thick.

12. A subcutaneous delivery system as in claim 1, wherein the matrix is an ethylene vinyl acetate (EVA) matrix; such as an EVA matrix having a vinyl acetate content of 28% to 40% and an ethylene content of 60% to 72%; preferably wherein the coating is cross-linked polydimethylsiloxane.

13. A subcutaneous delivery system as in claim1 comprising:
i) a biocompatible thermoplastic polyurethane matrix,
ii) an opioid embedded homogeneously in said matrix, and
iii) a biocompatible opioid impermeable cross-linked silicone polymer coating said matrix,
wherein said delivery system has a geometry such that there is an external coated wall and an internal uncoated wall forming an opening for release of said opioid, and the distance between the uncoated wall and the coated wall opposite the uncoated wall is substantially constant throughout the delivery system.

14. A subcutaneous delivery system as defined in claim 8 for use in providing prolonged relief of pain in a mammal suffering from pain.

15. A method of producing a subcutaneous implant as in claim 1 comprising the steps of:
i) forming a matrix polymer sheet of a first thermoplastic polymeric resin with a therapeutic agent dispersed in said matrix,
ii) die cutting said sheet to form polymer matrix, and
iii) coating said polymer matrix with an uncured silicone material which after curing is impermeable to said therapeutic agent.

16. A method as in claim 15 wherein silicone is a silicone dispersion or a silicone adhesive;
or wherein step i) is by solution casting;
or wherein after step iii) is the step of forming a channel in the coated polymer matrix;
or wherein the step iii) coating is done by solution coating.

17. A method as in claim 15 wherein after step iii) is the step of iv) coating the implant with a second thermoplastic resin; preferably wherein said first and/or said second thermoplastic polymeric resin is a resin blend or wherein said first thermoplastic polymeric resin and said second thermoplastic polymeric resin are the same; and optionally wherein the step iv) coating is done by hot melt extrusion or by powder coating and then thermal fusion.

18. A method as in claim 15 wherein more than one coating is applied to said polymer matrix, preferably wherein an outer coating is a second thermoplastic or silicone polymeric matrix containing a second therapeutic agent.

19. A method of producing a subcutaneous implant delivery system as in claim 1 comprising the steps of:
i) solution casting of a first thermoplastic polymeric elastomer resin with an opioid dispersed therein to form a polymer matrix in a cylindrical shape,
ii) solution coating polymeric silicone resin on said polymer matrix to form an opioid impermeable coating, and
iii) forming an uncoated channel in said implant.

20. A method of producing a subcutaneous implant as in claim 1 comprising the steps of:
i) mixing a first thermoplastic elastomer polymeric resin with a polar solvent to form a polymer solution,
ii) adding an therapeutic agent to the solution,
iii) introducing the solution into a mold,
iv) drying the solution to form a matrix, and
v) coating the matrix with a silicone adhesive or dispersion which is impermeable to the therapeutic agent.

21. A method as in claim 20 wherein said silicone dispersion or silicone adhesive comprises a polyorganosiloxane;
or wherein said first thermoplastic elastomer polymeric resin is a polyurethane elastomer, a copolyester elastomer, a polyether block amide elastomer or an ethylene vinyl acetate copolymer;
or wherein said drying step is done in such a way as to eliminate the polar solvent;
or wherein the polar solvent is DMF or methylene chloride;
or wherein the therapeutic agent is hydromorphone.

22. A method as is claim 20 wherein after step v) is the step of vi) coating the implant with a second thermoplastic elastomer polymeric resin selected from the group consisting of a polyurethane, copolyester or polyether block amide; preferably wherein said first thermoplastic polymeric elastomer resin and said second thermoplastic polymeric elastomer resin are the same.

## Patentansprüche

1. Subkutanes Abgabesystem, das Folgendes umfasst:
i) eine biokompatible thermoplastische Elastomermatrix,
ii) ein therapeutisches Mittel, das homogen in der genannten Matrix dispergiert ist, und
iii) ein biokompatibles für das therapeutische Mittel impermeables vernetztes Silikonpolymer, das die genannte Matrix beschichtet,
wobei das genannte Abgabesystem eine derartige Geometrie aufweist, dass eine äußere beschichtete Wand und eine innere unbeschichtete Wand vorhanden ist, die eine Öffnung für die Freisetzung des genannten therapeutischen Mittels bilden, und der Abstand zwischen der unbeschichteten Wand und der beschichteten Wand, die der unbeschichteten Wand gegenüberliegt, im gesamten Abgabesystem im Wesentlichen konstant ist.

2. Subkutanes Abgabesystem nach Anspruch 1, wobei das genannte Abgabesystem eine zylindrische Form aufweist.

3. Subkutanes Abgabesystem nach Anspruch 1, wobei die genannte Matrix eine Polyurethanmatrix ist; vorzugsweise wobei die genannte Urethanmatrix ein Isocyanat als hartes Segment und ein weiches PEG-, PPG- oder PTMEG-Glykolsegment aufweist.

4. Subkutanes Abgabesystem nach Anspruch 1, wobei die genannte Matrix eine Copolyestermatrix ist; wobei die genannte Copolyestermatrix vorzugsweise einen Polyester als hartes Segment und ein weiches PEG-, PPG- oder PTMEG-Glykolsegment aufweist.

5. Subkutanes Abgabesystem nach Anspruch 1, wobei die genannte Matrix eine Polyetherblockamidmatrix ist; bevorzugt, wobei die genannte Polyetherblockamidmatrix ein Polyamid als hartes Segment und ein weiches PEG-, PPG- oder PTMEG-Segment aufweist.

6. Subkutanes Abgabesystem nach Anspruch 3, 4 oder 5, wobei das harte Segment 20 bis 70 Gew.-% des Matrixpolymers bezogen auf den Rest des weichen Segments ausmacht; oder wobei etwa 50 % des therapeutischen Mittels in Lösung mit dem weichen Segment des Matrixpolymers vorliegen, während der restliche Teil des therapeutischen Mittels in der Matrix und nicht in Lösung dispergiert ist.

7. Subkutanes Abgabesystem nach Anspruch 1, wobei die genannte Matrix und Beschichtung nicht biologisch abbaubar sind; oder wobei das Silikon ein vernetztes Polyorganosiloxan ist; oder wobei das Silikon vernetztes Polydimethylsiloxan ist.

8. Subkutanes Abgabesystem nach Anspruch 1, wobei das genannte therapeutische Mittel ein Opioid, wie Buprenorphin, ist; oder wobei das genannte therapeutische Mittel aus der Gruppe ausgewählt wird, die aus Hydromorphon, Etorphin und Dihydroetorphin besteht; vorzugsweise wobei das genannte therapeutische Mittel Hydromorphon ist.

9. Subkutanes Abgabesystem nach Anspruch 1, wobei die genannte Matrix ein Polyurethan, wie ein Polyurethan auf Polyetherbasis, ist und die genannte Beschichtung vernetztes Polydimethylsiloxan ist.

10. Subkutanes Abgabesystem nach Anspruch 1, wobei die genannte Silikonpolymerbeschichtung eine Haftvermittlerschicht zwischen der genannten Polymermatrix und einer zweiten Schicht ist, die eine zweite biokompatible therapeutische Elastomermatrix umfasst.

11. Subkutanes Abgabesystem nach Anspruch 10, wobei die genannte zweite Schicht ein Copolyester, ein Polyetherblockamid oder ein thermoplastisches Polyurethan ist; oder wobei die genannte zweite Schicht ein zweites therapeutisches Mittel umfasst; oder wobei jede Beschichtung 24 bis 48 Mikron dick ist.

12. Subkutanes Abgabesystem nach Anspruch 1, wobei die Matrix eine Ethylenvinylacetat- (EVA) Matrix ist; wie eine EVA-Matrix mit einem Vinylacetatgehalt von 28 % bis 40 % und einem Ethylengehalt von 60 % bis 72 %; vorzugsweise wobei die Beschichtung vernetztes Polydimethylsiloxan ist.

13. Subkutanes Abgabesystem nach Anspruch 1, das Folgendes umfasst:
i) eine biokompatible thermoplastische Polyurethanmatrix,
ii) ein Opioid, das homogen in die genannte Matrix eingebettet ist, und
iii) ein biokompatibles Opioid-undurchlässiges vernetztes Silikonpolymer, das die genannte Matrix beschichtet,
wobei das genannte Abgabesystem eine derartige Geometrie aufweist, dass eine äußere beschichtete Wand und eine innere unbeschichtete Wand eine Öffnung für die Freisetzung des genannten Opioids bilden, und der Abstand zwischen der unbeschichteten Wand und der beschichteten Wand, die der unbeschichteten Wand gegenüberliegt, im gesamten Abgabesystem im Wesentlichen konstant ist.

14. Subkutanes Abgabesystem nach Anspruch 8 zur Verwendung bei der Bereitstellung einer verlängerten Schmerzlinderung bei einem Säuger, der an Schmerzen leidet.

15. Verfahren zur Herstellung eines subkutanen Implantats nach Anspruch 1, das folgende Schritte umfasst:
i) Bilden einer Matrixpolymerfolie aus einem ersten thermoplastischen Polymerharz mit einem therapeutischen Mittel, das in der genannten Matrix dispergiert ist,
ii) Stanzen der genannten Folie, um eine Polymermatrix zu bilden, und
iii) Beschichten der genannten Polymermatrix mit einem ungehärteten Silikonmaterial, das nach dem Aushärten für das genannte therapeutische Mittel undurchlässig ist.

16. Verfahren nach Anspruch 15, wobei Silikon eine Silikon-Dispersion oder ein Silikon-Klebstoff ist;
oder wobei Schritt i) durch Lösungsgießen erfolgt;
oder wobei nach Schritt iii) der Schritt des Bildens eines Kanals in der beschichteten Polymermatrix erfolgt;
oder wobei die Beschichtung von Schritt iii) durch Lösungsbeschichtung erfolgt.

17. Verfahren nach Anspruch 15, wobei nach Schritt iii) Schritt iv) des Beschichtens des Implantats mit einem zweiten thermoplastischen Harz erfolgt; vorzugsweise wobei das genannte erste und/oder das genannte zweite thermoplastische Polymerharz eine Harzmischung ist oder wobei das genannte erste thermoplastische Polymerharz und das genannte zweite thermoplastische Polymerharz gleich sind; und optional wobei die Beschichtung von Schritt iv) durch Heißschmelzextrusion oder Pulverbeschichtung und danach durch thermische Verschmelzung erfolgt.

18. Verfahren nach Anspruch 15, wobei mehr als eine Beschichtung vorzugsweise auf die genannte Polymermatrix aufgebracht wird, wobei eine äußere Beschichtung vorzugsweise eine zweite thermoplastische oder silikonpolymere Matrix ist, die ein zweites therapeutisches Mittel umfasst.

19. Verfahren zur Herstellung eines Systems zur Abgabe eines subkutanen Implantats nach Anspruch 1, wobei das Verfahren folgende Schritte umfasst:
i) Lösungsgießen eines ersten thermoplastischen polymeren Elastomerharzes mit einem darin dispergierten Opioid zur Bildung einer Polymermatrix in einer zylindrischen Form,
ii) Lösungsbeschichten von polymerem Silikonharz auf der genannten Polymermatrix, um eine opioidundurchlässige Beschichtung zu bilden, und
iii) Bilden eines unbeschichteten Kanals in dem genannten Implantat.

20. Verfahren zur Herstellung eines subkutanen Implantats nach Anspruch 1, das folgende Schritte umfasst:
i) Mischen eines ersten thermoplastischen Elastomerpolymerharzes mit einem polaren Lösungsmittel zur Bildung einer Polymerlösung,
ii) Zugabe eines therapeutischen Mittels zu der Lösung,
iii) Einführen der Lösung in eine Form,
iv) Trocknen der Lösung zur Bildung einer Matrix, und
v) Beschichten der Matrix mit einem Silikonkleber oder einer Dispersion, die für den therapeutischen Wirkstoff undurchlässig ist.

21. Verfahren nach Anspruch 20, wobei die genannte Silicon-Dispersion oder der Silikon-Klebstoff ein Polyorganosiloxan umfasst;
oder wobei das genannte erste thermoplastische Elastomerpolymerharz ein Polyurethanelastomer, ein Copolyesterelastomer, ein Polyetherblockamidelastomer oder ein Ethylenvinylacetatcopolymer ist;
oder wobei der genannte Trocknungsschritt in einer Weise durchgeführt wird, dass das polare Lösungsmittel eliminiert wird;
oder wobei das polare Lösungsmittel DMF oder Methylenchlorid ist;
oder wobei das therapeutische Mittel Hydromorphon ist.

22. Verfahren nach Anspruch 20, wobei nach Schritt v) Schritt vi) des Beschichtens des Implantats mit einem zweiten thermoplastischen Elastomerpolymerharz erfolgt, das aus der Gruppe ausgewählt wird, die aus einem Polyurethan, Copolyester oder Polyetherblockamid besteht; wobei das genannte erste thermoplastische polymere Elastomerharz und das genannte zweite thermoplastische polymere Elastomerharz vorzugsweise gleich sind.

## Revendications

1. Système d'administration sous-cutanée comprenant :
i) une matrice d'élastomère thermoplastique biocompatible,
ii) un agent thérapeutique dispersé de façon homogène dans ladite matrice, et
iii) un polymère de silicone réticulée biocompatible imperméable à l'agent thérapeutique revêtant ladite matrice,
ledit système d'administration ayant une géométrie telle qu'il existe une paroi revêtue externe et une paroi non revêtue interne formant une ouverture pour la libération dudit agent thérapeutique, et la distance entre la paroi non revêtue et la paroi revêtue opposée à la paroi non revêtue est sensiblement constante à travers le système d'administration.

2. Système d'administration sous-cutanée selon la revendication 1, ledit système d'administration étant de forme cylindrique.

3. Système d'administration sous-cutanée selon la revendication 1, dans lequel ladite matrice est une matrice de polyuréthane ; préférablement dans lequel ladite matrice d'uréthane comporte un isocyanate comme segment dur, et un PEG, un PPG ou un PTMEG glycol comme segment mou.

4. Système d'administration sous-cutanée selon la revendication 1, dans lequel ladite matrice est une matrice de copolyester ; préférablement dans lequel ladite matrice de copolyester comporte un polyester comme segment dur, et un PEG, un PPG ou un PTMEG glycol comme segment mou.

5. Système d'administration sous-cutanée selon la revendication 1, dans lequel ladite matrice est une matrice d'amide à blocs de polyéther ; préférablement dans lequel ladite matrice d'amide à blocs de polyéther comporte un polyamide comme segment dur, et un PEG, un PPG ou un PTMEG comme segment mou.

6. Système d'administration sous-cutanée selon la revendication 3, 4 ou 5, dans lequel le segment dur représente 20 à 70 % en poids du polymère de matrice, le reste étant constitué du segment mou ;
ou dans lequel environ 50 % de l'agent thérapeutique est en solution avec le segment mou du polymère de matrice tandis que le reste de l'agent thérapeutique est dispersé dans la matrice et n'est pas en solution.

7. Système d'administration sous-cutanée selon la revendication 1, dans lequel ladite matrice et ledit revêtement sont non biodégradables ; ou dans lequel la silicone est un polyorganosiloxane réticulé ; ou dans lequel la silicone est un polydiméthylsiloxane réticulé.

8. Système d'administration sous-cutanée selon la revendication 1, dans lequel ledit agent thérapeutique est un opioïde, tel que la buprénorphine ; ou dans lequel ledit agent thérapeutique est sélectionné dans le groupe constitué de l'hydromorphone, de l'étorphine et de la dihydroétorphine ; préférablement dans lequel ledit agent thérapeutique est l'hydromorphone.

9. Système d'administration sous-cutanée selon la revendication 1, dans lequel ladite matrice est un polyuréthane, tel qu'un polyuréthane à base de polyéther, et ledit revêtement est un polydiméthylsiloxane réticulé.

10. Système d'administration sous-cutanée selon la revendication 1, dans lequel ledit revêtement de polymère de silicone est une couche de liaison adhésive entre ladite matrice de polymère et une deuxième couche comprenant une deuxième matrice d'élastomère thérapeutique biocompatible.

11. Système d'administration sous-cutanée selon la revendication 10, dans lequel ladite deuxième couche est un copolyester, un amide à blocs de polyéther, ou un polyuréthane thermoplastique ;
ou dans lequel ladite deuxième couche contient un deuxième agent thérapeutique ;
ou dans lequel chaque couche a une épaisseur de 24 à 48 microns.

12. Système d'administration sous-cutanée selon la revendication 1, dans lequel la matrice est une matrice d'éthylène acétate de vinyle (EVA) ; telle qu'une matrice d'EVA ayant une teneur en acétate de vinyle de 28 % à 40 % et une teneur en éthylène de 60 % à 72 % ; préférablement dans lequel le revêtement se compose de polydiméthylsiloxane réticulé.

13. Système d'administration sous-cutanée selon la revendication 1, comprenant :
i) une matrice de polyuréthane thermoplastique biocompatible,
ii) un opioïde incorporé de façon homogène dans ladite matrice, et
iii) un polymère de silicone réticulée biocompatible imperméable à l'opioïde revêtant ladite matrice,
ledit système d'administration ayant une géométrie telle qu'il existe une paroi revêtue externe et une paroi non revêtue interne formant une ouverture pour la libération dudit opioïde, et la distance entre la paroi non revêtue et la paroi revêtue opposée à la paroi non revêtue est sensiblement constante à travers le système d'administration.

14. Système d'administration sous-cutanée tel que défini dans la revendication 8, destiné à une utilisation dans le soulagement prolongé de la douleur chez un mammifère souffrant de douleur.

15. Procédé de fabrication d'un implant sous-cutané selon la revendication 1, comprenant les étapes qui consistent à :
i) former une feuille de polymère de matrice se composant d'une première résine polymère thermoplastique, un agent thérapeutique étant dispersé dans ladite matrice,
ii) découper ladite feuille à l'emporte-pièce pour former une matrice de polymère, et
iii) revêtir ladite matrice de polymère avec un matériau silicone non durci qui, après durcissement, est imperméable audit agent thérapeutique.

16. Procédé selon la revendication 15, dans lequel la silicone est une dispersion de silicone ou un adhésif à base de silicone ;
ou dans lequel l'étape i) est réalisée par coulée en solution ;
ou dans lequel après l'étape iii) a lieu une étape de formation d'un canal dans la matrice de polymère revêtue ;
ou dans lequel le revêtement à l'étape iii) est réalisé par revêtement en solution.

17. Procédé selon la revendication 15, dans lequel après l'étape iii) a lieu une étape iv) consistant à revêtir l'implant d'une deuxième résine thermoplastique ; préférablement dans lequel ladite première et/ou ladite deuxième résine polymère thermoplastique sont un mélange de résines ou dans lequel ladite première résine polymère thermoplastique et ladite deuxième résine polymère thermoplastique sont identiques ; et optionnellement dans lequel le revêtement à l'étape iv) est réalisé par extrusion à chaud à l'état fondu ou par application d'un revêtement de poudre suivie d'une fusion thermique.

18. Procédé selon la revendication 15, dans lequel plus d'un revêtement est appliqué sur ladite matrice de polymère, préférablement dans lequel un revêtement externe est une deuxième matrice de polymère thermoplastique ou de silicone contenant un deuxième agent thérapeutique.

19. Procédé de fabrication d'un système d'administration sous la forme d'un implant sous-cutané selon la revendication 1, comprenant les étapes qui consistent à :
i) couler en solution une première résine polymère d'élastomère thermoplastique dans laquelle est dispersé un opioïde pour former une matrice de polymère de forme cylindrique,
ii) appliquer par revêtement en solution une résine polymère de silicone sur ladite matrice de polymère pour former un revêtement imperméable à l'opioïde, et
iii) former un canal non revêtu dans ledit implant.

20. Procédé de fabrication d'un implant sous-cutané selon la revendication 1, comprenant les étapes qui consistent à :
i) mélanger une première résine polymère d'élastomère thermoplastique avec un solvant polaire pour former une solution de polymère,
ii) ajouter un agent thérapeutique à la solution,
iii) introduire la solution dans un moule,
iv) sécher la solution pour former une matrice, et
v) revêtir la matrice d'un adhésif à base de silicone ou d'une dispersion de silicone qui est imperméable à l'agent thérapeutique.

21. Procédé selon la revendication 20, dans lequel ladite dispersion de silicone ou ledit adhésif à base de silicone comprend un polyorganosiloxane ;
ou dans lequel ladite première résine polymère d'élastomère thermoplastique est un élastomère de polyuréthane, un élastomère de copolyester, un élastomère d'amide à blocs de polyéther ou un copolymère d'éthylène acétate de vinyle ;
ou dans lequel ladite étape de séchage est réalisée de façon à éliminer le solvant polaire ;
ou dans lequel ledit solvant polaire est le DMF ou le chlorure de méthylène ;
ou dans lequel l'agent thérapeutique est l'hydromorphone.

22. Procédé selon la revendication 20, dans lequel après l'étape v) a lieu une étape vi) consistant à revêtir l'implant d'une deuxième résine polymère d'élastomère thermoplastique sélectionnée dans le groupe constitué d'un polyuréthane, d'un copolyester ou d'un amide à blocs de polyéther ; préférablement dans lequel ladite première résine polymère d'élastomère thermoplastique et ladite deuxième résine polymère d'élastomère thermoplastique sont identiques.
